# EUROPEAN PATENT APPLICATION

(11) **EP 4 537 890 A1**
(43) Date of publication of application: **16.04.2025**
(21) Application number: 23818882.5
(22) Date of filing: 11.05.2023
(51) Int. Cl.: A61M 60/135

(54) **DRIVING DEVICE AND BLOOD PUMP**

(30) Priority: 10.06.2022 CN 202210654824; 10.06.2022 CN 202210654851
(71) Applicant: Shenzhen Core Medical Technology Co., Ltd., Shenzhen, Guangdong 518000 (CN)
(72) Inventor: XIE, Duanqing, Shenzhen, Guangdong 518000 (CN); YU, Shunzhou, Shenzhen, Guangdong 518000 (CN)
(74) Representative: Murgitroyd & Company
(86) International application number: PCT/CN2023/093445
(87) International publication number: WO 2023/236717

(57) **Abstract**

Provided are a driving device (400) and a blood pump (10). The driving device (400) comprises a pump housing (410), a stator (430), a rotor (440), a wire (450), and a cover (470). The pump housing (410) comprises a first wiring through-hole (413), a second wiring through-hole (414), and a limiting groove (416d). The limiting groove (416d) is located on the outer side of the housing wall of the pump housing (410). The wire (450) passes through the first wiring through-hole (413) and the second wiring through-hole (414). The wire (450) comprises an isolation section (452) corresponding to the position of the rotor (440). The isolation section (452) is located between the first wiring through-hole (413) and the second wiring through-hole (414). At least part of the isolation section (452) is limited in the limiting groove (416d). The cover (470) shields the first wiring through-hole (413), the second wiring through-hole (414), the limiting groove (416d), and the isolation section (452).

## Description

This application claims priority to Chinese Patent Applications No. CN202210654824.5 and No. CN202210654851.2 filed with the China Patent Office on June 10, 2022, the entire contents of which are incorporated herein by reference.

### TECHNICAL FIELD

The present application relates to the field of medical device technologies, and in particular, to a driving device and a blood pump.

### BACKGROUND

An intravascular blood pump is a pumping device that can be inserted into a patient's heart through the patient's blood vessels. The intravascular blood pump is placed inside an opening of a heart valve so that blood can flow through the blood pump and into arterial blood vessels. However, traditional blood pumps suffer from a high failure rate.

### SUMMARY

Accordingly, the present application provides a driving device and a blood pump, which can reduce a failure rate of the blood pump.

In a first aspect of the present application, embodiments provide a driving device configured to drive an impeller to rotate. The driving device includes:
a pump case having an accommodation cavity, a casing wall of the pump case being further provided with a first wire hole, a second wire hole, and a limiting groove, the second wire hole and the first wire hole being in communication with the accommodation cavity, the second wire hole being spaced apart from the first wire hole, the limiting groove being located on an outer side of the casing wall of the pump case, and the limiting groove being located between the first wire hole and the second wire hole;
a rotating shaft configured to be connected to the impeller, the rotating shaft being rotatably mounted on the pump case;
a rotor rotatably received in the accommodation cavity, the rotor being fixedly connected to the rotating shaft, and the rotor being capable of driving the rotating shaft to rotate;
a stator received in the accommodation cavity, the stator being capable of driving the rotor to rotate;
a conducting wire connected to the stator, the conducting wire extending through the first wire hole and the second wire hole, the conducting wire having an isolation section corresponding to a position of the rotor, the isolation section being located between the first wire hole and the second wire hole, the isolation section being at least partially limited in the limiting groove, and a groove wall of the limiting groove being located between the rotor and the isolation section; and
a cover connected to the pump case and completely covering the first wire hole, the second wire hole, the limiting groove, and the isolation section.

In a second aspect of the present application, embodiments provide a blood pump, including an impeller and a driving device. The driving device is configured to drive an impeller to rotate. The driving device includes:
a pump case having an accommodation cavity, a casing wall of the pump case being further provided with a first wire hole, a second wire hole, and a limiting groove, the second wire hole and the first wire hole being in communication with the accommodation cavity, the second wire hole being spaced apart from the first wire hole, the limiting groove being located on an outer side of the casing wall of the pump case, and the limiting groove being located between the first wire hole and the second wire hole;
a rotating shaft configured to be connected to the impeller, the rotating shaft being rotatably mounted on the pump case;
a rotor rotatably received in the accommodation cavity, the rotor being fixedly connected to the rotating shaft, and the rotor being capable of driving the rotating shaft to rotate;
a stator received in the accommodation cavity, the stator being capable of driving the rotor to rotate;
a conducting wire connected to the stator, the conducting wire extending through the first wire hole and the second wire hole, the conducting wire having an isolation section corresponding to a position of the rotor, the isolation section being located between the first wire hole and the second wire hole, the isolation section being at least partially limited in the limiting groove, and a groove wall of the limiting groove being located between the rotor and the isolation section; and
a cover connected to the pump case and completely covering the first wire hole, the second wire hole, the limiting groove, and the isolation section;
wherein the impeller is fixedly connected to the rotating shaft, and the impeller is rotatable with the rotating shaft.

Details of one or more embodiments of the present application are set forth in the following accompanying drawings and descriptions. Other features, objectives, and advantages of the present application become obvious with reference to the specification, the accompanying drawings, and the claims.

### BRIEF DESCRIPTION OF THE DRAWINGS

In order to more clearly illustrate the technical solutions in embodiments of the present application, the accompanying drawings used in the description of the embodiments or the prior art will be briefly introduced below. It is apparent that, the accompanying drawings in the following description are only for some embodiments of the present application, and other drawings can be obtained by those of ordinary skill in the art from the provided drawings without creative efforts.
FIG. 1 is a schematic view of a three-dimensional structure of a blood pump according to an embodiment;
FIG. 2 is a schematic view of a plane structure of the blood pump shown in FIG. 1 with a catheter and part of a cannula assembly omitted;
FIG. 3 is a schematic view of a sectional structure of FIG. 2 in a first direction;
FIG. 4 is a schematic view of a sectional structure of FIG. 2 in a second direction;
FIG. 5 is a schematic view of a three-dimensional structure of a first stator member in a driving device of the blood pump shown in FIG. 1;
FIG. 6 is a schematic view of the first stator member shown in FIG. 5 from another perspective;
FIG. 7 is a schematic structural view of a rotor in the driving device of the blood pump shown in FIG. 1;
FIG. 8 is a sectional view of the rotor shown in FIG. 7;
FIG. 9 is a schematic view of an exploded structure of the rotor shown in FIG. 7;
FIG. 10 is a three-dimensional exploded view of FIG. 2 with the cannula assembly completely omitted;
FIG. 11 is another three-dimensional exploded view of FIG. 2 with the cannula assembly completely omitted;
FIG. 12 is another three-dimensional exploded view of FIG. 2 with the cannula assembly completely omitted;
FIG. 13 is a sectional view of a pump case of the driving device of the blood pump in FIG. 1;
FIG. 14 is an exploded view of the pump case of the driving device of the blood pump in FIG. 1;
FIG. 15 is a schematic structural view of a second casing of the pump case shown in FIG. 14;
FIG. 16 is a schematic view of a three-dimensional structure of a first shaft sleeve of a shaft sleeve assembly of the blood pump shown in FIG. 3;
FIG. 17 is a schematic diagram of a three-dimensional structure of a second shaft sleeve of the shaft sleeve assembly of the blood pump shown in FIG. 3;
FIG. 18 is a sectional view of the shaft sleeve assembly of the blood pump shown in FIG. 3; and
FIG. 19 is an enlarged view of Part A of the blood pump shown in FIG. 4.

### DETAILED DESCRIPTION

In order to make the objects, technical solutions, and advantages of the present application clearer, the present application will be further described in detail below with reference to the accompanying drawings and embodiments. It should be understood that specific embodiments described herein are only intended to explain the present application and are not intended to limit the present application.

It is to be noted that when one element is referred to as being "fixed to" or "disposed on" another element, it may be directly disposed on the another element or indirectly disposed on the another element. When one element is considered to be "connected to" another element, it may be directly connected to the another element or indirectly connected to the another element.

In addition, the terms "first" and "second" are used for descriptive purposes only, which cannot be construed as indicating or implying a relative importance, or implicitly specifying the number of the indicated technical features. Thus, the features defined by "first" and "second" may explicitly or implicitly include one or more features. In the description of the present application, "a plurality of" means two or more, unless otherwise defined explicitly and specifically.

In order to illustrate the technical solutions of the present application, description will be provided below with reference to specific drawings and embodiments.

Herein, "proximal end" is defined as an end adjacent to an operator, and "distal end" is defined as an end away from the operator.

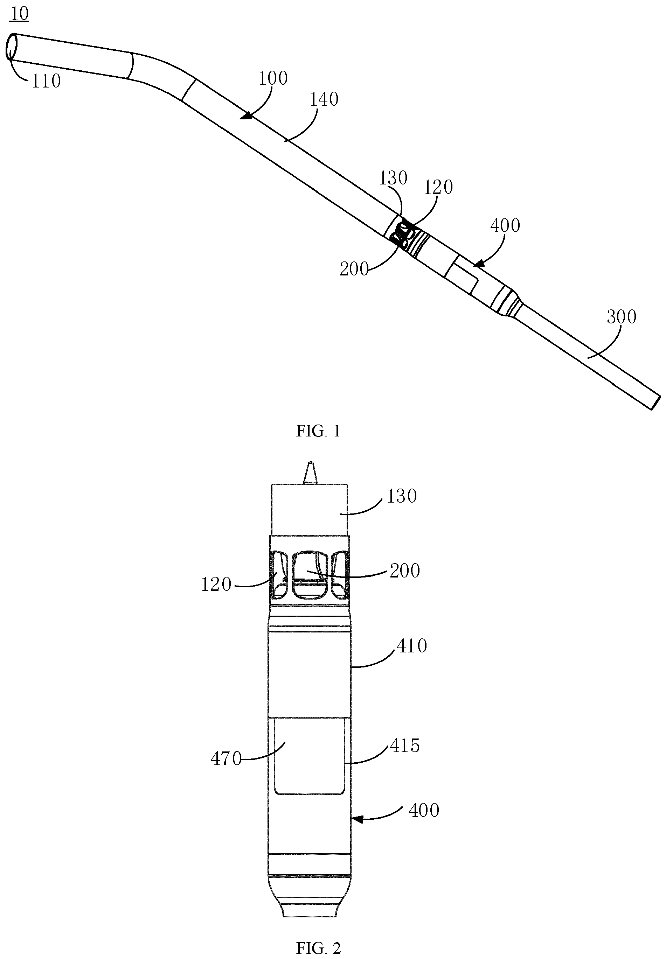

Referring to FIG. 1 and FIG. 2, a blood pump 10 according to an embodiment of the present application includes a cannula assembly 100, an impeller 200, a catheter 300, and a driving device 400. The cannula assembly 100 is connected to an end of the driving device 400. The catheter 300 is connected to another end of the driving device 400. The impeller 200 is rotatably disposed in the cannula assembly 100. The impeller 200 is connected to the driving device 400. The driving device 400 can drive the impeller 200 to rotate to realize a pumping function of the blood pump 10.

Specifically, the cannula assembly 100 has an inlet 110 and an outlet 120. The outlet 120 is closer to the driving device 400 than the inlet 110. In an embodiment, the cannula assembly 100 extends through a heart valve, such as an aortic valve. The inlet 110 is located within the heart, and the outlet 120 and the driving device 400 are located in a blood vessel outside the heart such as an aorta. When the impeller 200 rotates, blood flows into the cannula assembly 100 via the inlet 110 and flows out of the cannula assembly 100 via the outlet 120.

In the illustrated embodiments, the cannula assembly 100 includes a first cannula 130 and a second cannula 140 connected to the first cannula 130. An end of the first cannula 130 away from the second cannula 140 is connected to the driving device 400. The inlet 110 is provided on the second cannula 140, and the outlet 120 is provided on the first cannula 130.

A position of the impeller 200 generally corresponds to a position of the outlet 120. Specifically, the impeller 200 is located in the first cannula 130.

The catheter 300 is connected to an end of the driving device 400 away from the cannula assembly 100. The catheter 300 is configured to house various supply lines. The supply lines may be, for example, a cleaning line configured to introduce cleaning fluid into the driving device 400, a conducting wire supplying power to the driving device 400, a supporting component configured to support the catheter 300, or the like.

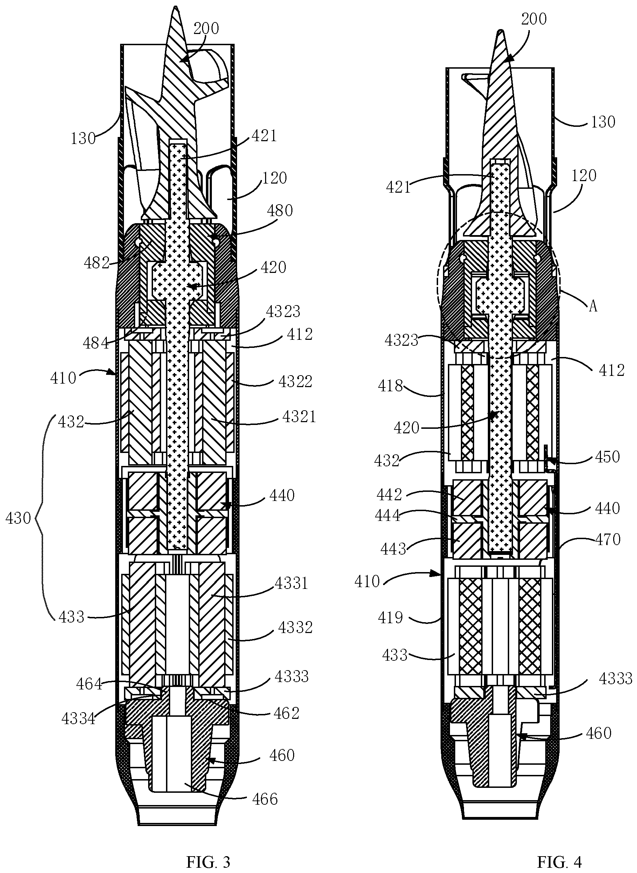

Referring to FIG. 3 to FIG. 4, the driving device 400 includes a pump case 410, a rotating shaft 420, a stator 430, a rotor 440, and a conducting wire 450. The rotating shaft 420 is rotatably mounted on the pump case 410. The stator 430 and the rotor 440 are both received in the pump case 410. The rotor 440 is fixedly connected to the rotating shaft 420. At least part of the conducting wire 450 is received in the pump case 410. The conducting wire 450 is connected to the stator 430 to supply power to the stator 430. The stator 430 can drive the rotor 440 to rotate, the rotor 440 can drive the rotating shaft 420 to rotate, and the impeller 200 can rotate along with the rotating shaft 420.

In some embodiments, the pump case 410 is generally in the shape of a cylinder with a circular cross-section. The pump case 410 has an accommodation cavity 412.

The rotating shaft 420 has an end received in the accommodation cavity 412 and another end extending out of the pump case 410 and fixedly connected to the impeller 200. The rotating shaft 420 extends in the same direction as an axial direction of the pump case 410. The end of the rotating shaft 420 configured to be connected to the impeller 200 is a connecting end 421. The rotating shaft 420 may be made of a material such as ceramic or stainless steel. For example, the ceramic is alumina toughened zirconia (ATZ) ceramic, and the stainless steel is SUS316L. In this way, torsional strength of the rotating shaft 420 can be improved, preventing breakage of the rotating shaft 420 due to excessive torque.

The stator 430 is received in the accommodation cavity 412. In the illustrated embodiments, the stator 430 includes a first stator member 432 and a second stator member 433. Both the first stator member 432 and the second stator member 433 can drive the rotor 440 to rotate. Specifically, the first stator member 432 and the second stator member 433 are spaced apart in the extending direction of the rotating shaft 420. Both the first stator member 432 and the second stator member 433 are fixedly connected to the pump case 410. The rotating shaft 420 rotatably extends through the first stator member 432. That is, the rotating shaft 420 is rotatable relative to the pump case 410, while the first stator member 432 and the second stator member 433 are non-rotatable relative to the pump case 410.

The first stator member 432 and the second stator member 433 may be connected in parallel or in series. In some embodiments, the first stator member 432 and the second stator member 433 can synchronously drive the rotor 440 to rotate. The first stator member 432 and the second stator member 433 can jointly drive the rotor 440 to rotate or can separately drive the rotor 440 to rotate.

In some embodiments, the rotor 440 is magnetic, and the stator 430 can generate a rotating magnetic field that drives the rotor 440 to rotate. Specifically, both the first stator member 432 and the second stator member 433 can generate a rotating magnetic field that drives the rotor 440 to rotate.

Specifically, the first stator member 432 includes a first magnetic core 4321, a first coil 4322, and a first back plate 4323. The first back plate 4323 is fixedly connected to the pump case 410. A plurality of first magnetic cores 4321 are provided. The plurality of first magnetic cores 4321 are spaced apart along a circumference. Specifically, each first magnetic core 4321 extends in the same direction as the extending direction of the rotating shaft 420. Each first magnetic core 4321 is fixedly connected to the first back plate 4323. The first coil 4322 is wound around the first magnetic core 4321. In the illustrated embodiments, the first coil 4322 is wound around each first magnetic core 4321.

The second stator member 433 has a configuration similar to that of the first stator member 432. The second stator member 433 includes a second magnetic core 4331, a second coil 4332, and a second back plate 4333. The second back plate 4333 is fixedly connected to the pump case 410. A plurality of second magnetic cores 4331 are provided. The plurality of second magnetic cores 4331 are spaced apart along a circumference. Specifically, an extending direction of each second magnetic core 4331 is parallel to an axis of the rotating shaft 420. Each second magnetic core 4331 is fixedly connected to the second back plate 4333. The second coil 4332 is wound around the second magnetic core 4331. In the illustrated embodiments, the second coil 4332 is wound around each second magnetic core 4331.

In some embodiments, the first magnetic core 4321 and the second magnetic core 4331 each include a magnetic post and a head (i.e., a pole shoe) disposed at an end of the magnetic post. The magnetic post extends in the same direction as the extending direction of the rotating shaft. The first back plate 4323 is engaged with an end of the magnetic post of the first magnetic core 4321 away from the head, and the second back plate 4333 is engaged with an end of the magnetic post of the second magnetic core 4331 away from the head. In the extending direction of the magnetic post, the magnetic post is roughly a cylindrical body with a uniform size. That is, the size of a cross section of the magnetic post 4331 remain constant. Generally, the magnetic post 4331 is uniform in thickness. The first coil 4322 is wound around the magnetic post of the first magnetic core 4321, and the second coil 4332 is wound around the magnetic post of the second magnetic core 4331.

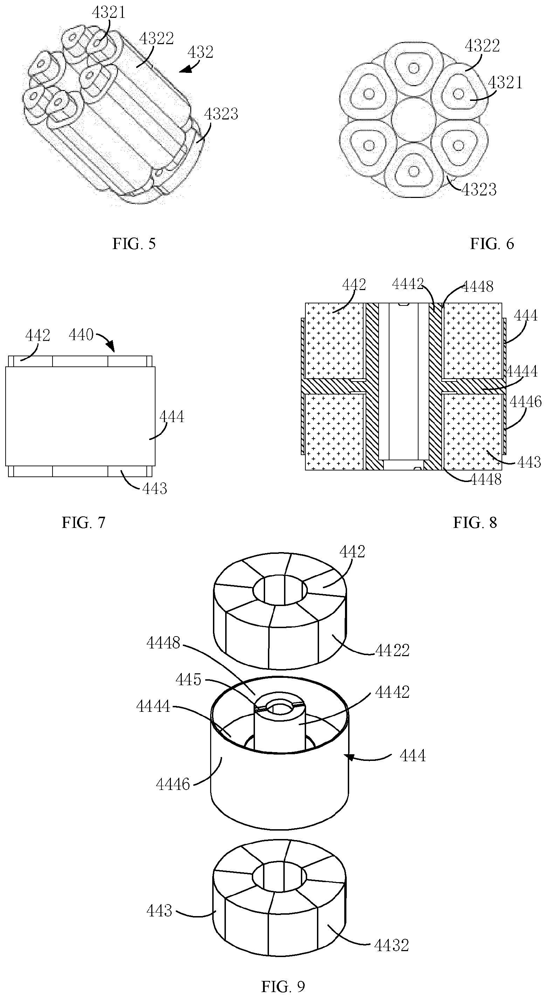

Referring to FIG. 5 and FIG. 6 together, in the illustrated embodiments, the first magnetic core 4321 and the second magnetic core 4331 each include only the magnetic post. That is, neither of the first magnetic core 4321 and the second magnetic core 4331 is provided with a wider head (i.e., pole shoe). In this case, the entire first magnetic core 4321 can be magnetically coupled with the rotor 440, and the entire second magnetic core 4331 can be magnetically coupled with the rotor 440. Compared with the magnetic core with the pole shoe, on the one hand, the magnetic core with only the magnetic post can reduce magnetic losses, increase magnetic coupling density between the magnetic core and the rotor, and increase torque of the stator member to the rotor under a same current. On the other hand, the magnetic core without a head can also greatly reduce the problem of power reduction of the driving device 400 caused by a local magnetic short circuit due to contact between adjacent magnetic cores.

It may be understood that the first magnetic core 4321 and the second magnetic core 4331 are not limited to have the above two configurations. In some embodiments, one of the first magnetic core 4321 and the second magnetic core 4331 has both the magnetic post and the head, and the other of the first magnetic core 4321 and the second magnetic core 4331 has only the magnetic post.

Cross sections of the magnetic posts of the first magnetic core 4321 and the second magnetic core 4331 may be in the shape of a sector, a circle, a trapezoid, a sector ring, or the like. In the illustrated embodiments, the magnetic post is generally in the shape of a triangular prism, and one edge of each magnetic post faces an axis of the rotating shaft. In some embodiments, edges of the magnetic post are all rounded, that is, the edges of the magnetic post are relatively smooth and blunt rounded edges, thereby eliminating sharp edges and corners on the magnetic post, which facilitates subsequent winding of the coil and also helps protect an insulating material covering the coil.

In the illustrated embodiments, in the extending direction of the rotating shaft 420, the rotating shaft 420 is spaced apart from the second stator member 433, that is, an end of the rotating shaft 420 away from the impeller 200 is spaced apart from the second stator member 433. That is, the rotating shaft 420 is not inserted into the second stator member 433. In other words, in the extending direction of the rotating shaft 420, an end of the rotating shaft 420 away from the connecting end 421 is spaced apart from the second stator member 433. The area of the cross section of the magnetic post of the second stator member 433 is greater than the area of the cross section of the magnetic post of the first stator member 432. In the illustrated embodiments, since the first magnetic core 4321 and the second magnetic core 4331 include only the magnetic posts, the magnetic post of the first stator member 432 is the first magnetic core 4321, and the magnetic post of the second stator member 433 is the second magnetic core 4331.

If the cross-sectional area of the magnetic post is larger, greater magnetic flux is generated, the torque of the stator member to the rotor 440 is relatively larger, and a smaller current is required, which helps reduce power consumption and reduce heat generation. In the case that sizes of cross sections of the first stator member 432 and the second stator member 433 are the same and an outer diameter of the pump case 410 remains unchanged, since the rotating shaft 420 is located outside the second stator member 433 and the rotating shaft 420 is not inserted into the second stator member 433, the size of the cross section of the magnetic post of the second stator member 433 can be reasonably increased without increasing the outer diameter of the pump case 410. In this way, driving torque of the second stator member 433 to the rotor 440 can be increased. With the same required torque, this can reasonably reduce the current supply to the coil of the stator, thereby reducing power consumption and also reducing an amount of heat generation of the driving device 400, which prevents discomfort or even harm to a human body caused by an excessively high temperature of the blood pump due to heat accumulation during operation.

It is to be noted that in other embodiments, the rotating shaft 420 may alternatively be inserted into the second stator member 433. In this case, the cross sections of the magnetic posts of the first stator member 432 and the second stator member 433 have the same area.

The first back plate 4323 and the second back plate 4333 generally have a flat platelike structure. The first back plate 4323 and the second back plate 4333 are made of a same material as the first magnetic core 4321 and the second magnetic core 4331, for example, a soft magnetic material such as cobalt steel.

The back plate can function as closing a magnetic circuit of the stator member to promote and increase the generation of magnetic flux in the stator member and improve a coupling capability between the stator member and the rotor 440. In other words, disposing the first back plate 4323 in the first stator member 432 can promote and increase the generation of magnetic flux in the first stator member 432 and improve the coupling capability between the first stator member 432 and the rotor 440, and disposing the second back plate 4333 in the second stator member 433 can promote and increase the generation of magnetic flux in the second stator member 433 and improve the coupling capability between the second stator member 433 and the rotor 440. Since the back plate can increase the magnetic flux, disposing the back plates in the first stator member 432 and the second stator member 433 respectively also helps reduce an overall diameter of the driving device 400.

Referring to FIG. 3 and FIG. 4 together, the driving device 400 further includes a positioning member 460. The positioning member 460 is fixedly connected in the pump case 410. The positioning member 460 has a bearing surface 462 and a positioning post 464. An end of the positioning post 464 is fixed to the bearing surface 462, and the other end of the positioning post 464 protrudes a certain height from the bearing surface 462. That is, the positioning post 464 is disposed on and protrudes from the bearing surface 462. The second back plate 4333 of the second stator member 433 is provided with a positioning hole 4334. The positioning post 464 is inserted into the positioning hole 4334. The second back plate 4333 abuts against the bearing surface 462. In this way, the positioning member 460 can position the second stator member 433, thereby improving mounting accuracy and mounting efficiency of the second stator member 433. Specifically, a central axis of the positioning post 464 and a central axis of the second stator member 433 coincide with each other.

The positioning member 460 is further provided with a through hole 466. The through hole 466 may be used to be in communication with a cleaning line introducing cleaning fluid into the driving device 400, or a cleaning line is mounted in the through hole 466.

It is to be noted that in some embodiments, the first back plate 4323 of the first stator member 432 may be omitted, and the second back plate 4333 of the second stator member 433 may be omitted. Alternatively, one of the first stator member 432 and the second stator member 433 has a back plate, and the other of the first stator member 432 and the second stator member 433 has no back plate. If the second back plate 4333 of the second stator member 433 is omitted, a plurality of positioning holes may be directly provided in the positioning member 460. One end of each of the plurality of second magnetic cores 4331 is positioned in the respective one of the plurality of positioning holes.

In some embodiments, the positioning member 460 may be omitted. In this case, an engagement element for engaging with an edge of the second back plate 4333 may be disposed in the pump case 410, so that the second stator member 433 can be fixed by engaging the engagement element with the second back plate 4333. Alternatively, the second stator member 433 is bonded and fixed to the pump case 410 through an adhesive. The first stator member 432 may be bonded and fixed to the pump case 410 through an adhesive, or may be engaged with the first back plate 4323 by disposing a corresponding engagement element in the pump case 410, to achieve fixing of the first stator member 432.

The rotor 440 is received in the accommodation cavity 412 of the pump case 410. Specifically, the rotor 440 includes a first magnet 442 and a second magnet 443. The first magnet 442 and the second magnet 443 are both fixedly connected to the rotating shaft 420. The second magnet 443 is located between the first stator member 432 and the second stator member 433. The first stator member 432 can generate a rotating magnetic field that drives the first magnet 442 to rotate. The second stator member 433 can generate a rotating magnetic field that drives the second magnet 443 to rotate. The two stator members respectively provide torque to the rotor 440 through the two magnets, which can increase a driving force for the rotation of the rotor 440.

In the illustrated embodiments, the first magnet 442 and the second magnet 443 are both located between the first stator member 432 and the second stator member 433. That is, in the extending direction of the rotating shaft 420, the first stator member 432, the first magnet 442, the second magnet 443, and the second stator member 433 are arranged in sequence.

Specifically, the rotor 440 further includes a flywheel 444. The flywheel 444 is fixedly connected to the rotating shaft 420. The flywheel 444 is located between the first stator member 432 and the second stator member 433. The first magnet 442 and the second magnet 443 are both fixedly connected to the flywheel 444. More specifically, the flywheel 444 is fixedly connected to an end of the rotating shaft 420 away from the connecting end 421.

Through the arrangement of the flywheel 444, strength of connection between the first magnet 442 and the second magnet 443 and the rotating shaft 420 can be enhanced. In addition, by disposing both the first magnet 442 and the second magnet 443 on the same flywheel 444, shaking of the rotating shaft 420 during the rotation can be reduced, so that the rotating shaft 420 is more stable during the rotation.

Referring to FIG. 7 to FIG. 9, in the illustrated embodiments, the flywheel 444 includes a built-in tube 4442, a disc-shaped portion 4444, and an outer annular wall 4446. Both the built-in tube 4442 and the outer annular wall 4446 have a circular tube structure, and the disc-shaped portion 4444 has an annular disc structure. Both the built-in tube 4442 and the outer annular wall 4446 are fixedly connected to the disc-shaped portion 4444. The outer annular wall 4446 is disposed around the disc-shaped portion 4444. The built-in tube 4442 and the outer annular wall 4446 are coaxially disposed. The rotating shaft 420 extends through the built-in tube 4442 and is fixedly connected to the built-in tube 4442. An accommodation space is formed between the built-in tube 4442 and the outer annular wall 4446. The disc-shaped portion 4444 divides the accommodation space into two mounting cavities 4448. The two mounting cavities 4448 are both annular. The first magnet 442 and the second magnet 443 are respectively accommodated in the two mounting cavities 4448. The first magnet 442 and the second magnet 443 are both annular, and shapes of the two mounting cavities 4448 match the first magnet 442 and the second magnet 443 respectively, to facilitate mounting and positioning of the first magnet 442 and the second magnet 443. In this way, the flywheel 444 can have a limiting effect on the first magnet 442 and the second magnet 443, which not only facilitates the mounting of the first magnet 442 and the second magnet 443, and also makes the combination of the first magnet 442 and the second magnet 443 with the flywheel 444 more stable.

It is to be noted that the flywheel 444 is not limited to have the above structure. In some embodiments, the outer annular wall 4446 of the flywheel 444 is omitted. In some embodiments, the outer annular wall 4446 and the built-in tube 4442 of the flywheel 444 are omitted. In this case, the rotating shaft 420 fixedly extends through the disc-shaped portion 4444, for example, extends through a center of the disc-shaped portion 4444. Compared with the flywheel 444 having only the disc-shaped portion 4444, through the arrangement of the built-in tube 4442, the flywheel 444 and the rotating shaft 420 can be connected more stably.

In some embodiments, the first magnet 442 and the second magnet 443 are both annular Halbach array magnets. Specifically, the first magnet 442 and the second magnet 443 each include a plurality of magnetic bodies, for example, four, six, eight, ten, or the like magnetic bodies may be provided. Each magnetic body is in the shape of a sector ring. The plurality of magnetic bodies of the first magnet are arranged around the rotating shaft 420 in a circle to form an annular structure, and the plurality of magnetic bodies of the second magnet 443 are arranged around the rotating shaft 420 in a circle to form an annular structure.

More specifically, the first magnet 442 has a first magnetic body 4422 magnetized in an axial direction of the first magnet 442; the second magnet 443 has a second magnetic body 4432 magnetized in an axial direction of the second magnet 443. The first magnetic body 4422 and the second magnetic body 4432 are respectively disposed on two opposite sides of the disc-shaped portion 4444, and positions of the first magnetic body 4422 and the second magnetic body 4432 correspond to each other. In the extending direction of the rotating shaft 420, the first magnetic body 4422 and the second magnetic body 4432 have opposite polarities on a respective side thereof facing the disc-shaped portion 4444. In this way, the mounting of the first magnet 442 and the second magnet 443 can be facilitated, and the problem of assembling difficulties caused by mutual repulsion between the first magnetic body 4422 of the first magnet 442 corresponding to the disc-shaped portion 4444 and the second magnetic body 4432 of the second magnet 443 corresponding to the disc-shaped portion 4444 is prevented. For example, if the first magnetic body 4422 has N-pole polarity on the side facing the disc-shaped portion 4444, the polarity of the second magnetic body 4432 has S-pole polarity on the side facing the disc-shaped portion 4444. According to the principle of mutual attraction between the N pole and the S pole, interference of magnetic repulsion is eliminated, and mounting efficiency of the first magnet 442 and the second magnet 443 is improved.

To facilitate the mounting of the first magnet 442 and the second magnet 443 and improve the mounting accuracy of the first magnet 442 and the second magnet 443, the flywheel 444 is further provided with an identification portion 445 configured to determine a mounting position of the first magnetic body 4422 and a mounting position of the second magnetic body 4432. The identification portion 445 may be provided as a groove, a scale line, an identification, or the like. When the first magnet 442 and the second magnet 443 are mounted, as long as the identification portion 445 is used to identify the position of the magnetic body of one of the first magnet 442 and the second magnet 443, mounting positions of the remaining magnetic bodies may be determined, so as to facilitate the mounting of the first magnet 442 and the second magnet 443. Specifically, the identification portion 445 is disposed on at least one of the built-in tube 4442, the disc-shaped portion 4444, and the outer annular wall 4446. Specifically, in the illustrated embodiments, the identification portions 445 are disposed on end surfaces of two ends of the built-in tube 4442 respectively.

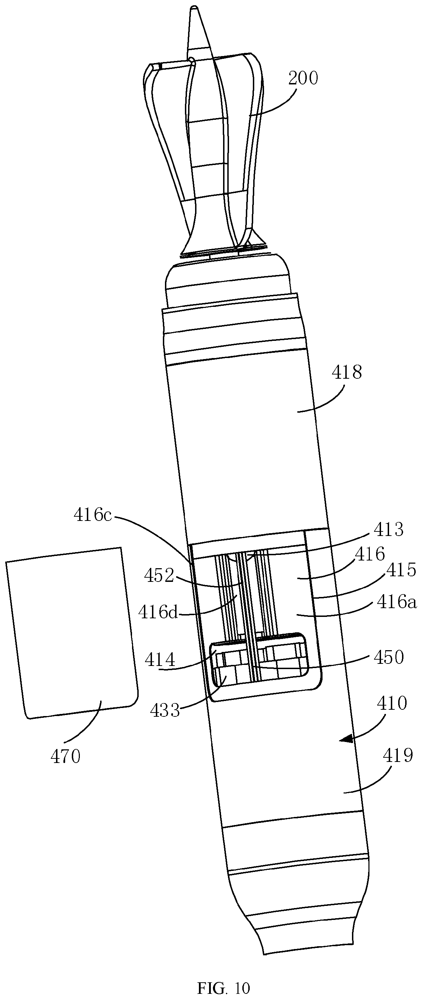

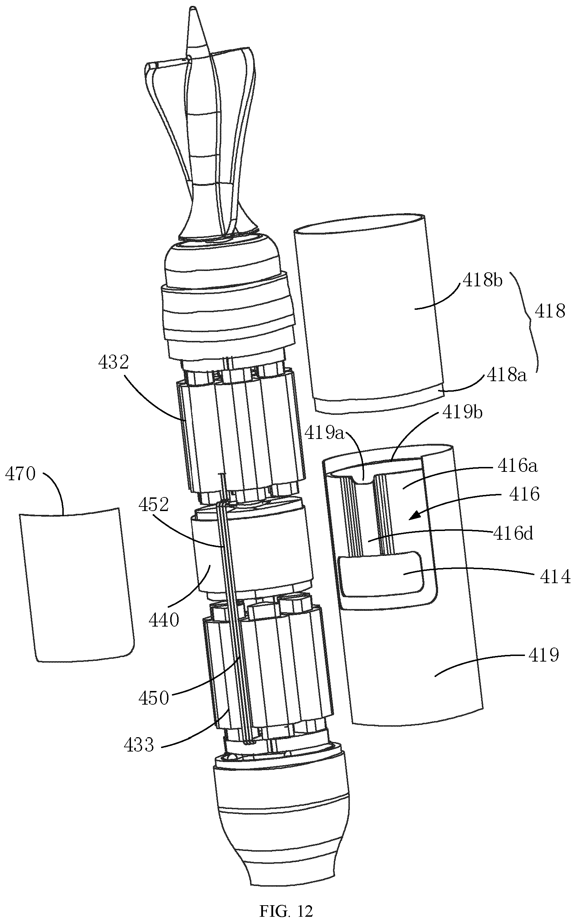

Referring to FIG. 4 and FIG. 10 to FIG. 12, in the illustrated embodiments, the conducting wire 450 has an end connected to the first stator member 432 and another end connected to the second stator member 433. In some embodiments, the two ends of the conducting wire 450 are respectively electrically connected to the first coil 4322 of the first stator member 432 and the second coil 4223 of the second stator member 433. It may be understood that the connection manner between the first stator member 432 and the second stator member 433 is not limited to the above. In some embodiments, an end of the conducting wire 450 away from the first stator member 432 extends from the pump case 410 into the catheter 300 and is connected to an external power supply device, and the second stator member 433 is connected to the external power supply device through another conducting wire.

Since the rotor 440 is disposed between the first stator member 432 and the second stator member 433, regardless of whether two ends of the conducting wire 450 are connected to the first stator member 432 and the second stator member 433 respectively or the conducting wire 450 has an end connected to the first stator member 432 and another end extending from the pump case 410 into the catheter 300, the conducting wire 450 has a section corresponding to the position of the rotor 440. A portion of the conducting wire 450 corresponding to the position of the rotor 440 is defined as the isolation section 452. If the rotor 440 comes into contact with the isolation section 452 during the rotation, there is a risk of failures such as breakage or falling-off of the conducting wire 450 caused by rotation of the isolation section 452 along with the rotor 440.

To this end, in this embodiment, the casing wall of the pump case 410 is further provided with a first wire hole 413 and a second wire hole 414 that are spaced apart. The first wire hole 413 and the second wire hole 414 are both in communication with the accommodation cavity 412. The conducting wire 450 extends through the first wire hole 413 and the second wire hole 414. The isolation section 452 is located between the first wire hole 413 and the second wire hole 414. The isolation section 452 is located outside the accommodation cavity 412. That is, since the rotor 440 is located in the accommodation cavity 412, the rotor 440 and the isolation section 452 of the conducting wire 450 are respectively located on the two sides of the casing wall of the pump case 410 between the first wire hole 413 and the second wire hole 414. In this way, the isolation section 452 of the conducting wire 450 can be separated from the rotor 440, thereby effectively preventing failures of breakage or falling-off of the conducting wire 450 caused by rotation of the conducting wire 450 along with the rotor 440 due to the rotor 440 contacting with the conducting wire 450 during the rotation, thereby ensuring normal use of the blood pump 10.

In order to prevent entry of foreign objects (such as blood) into the accommodation cavity 412 through the first wire hole 413 and the second wire hole 414 and also prevent exposure of the isolation section 452 of the conducting wire 450, the driving device 400 further includes a cover 470. The cover 470 is connected to the pump case 410. The cover 470 completely covers the first wire hole 413, the second wire hole 414, and the isolation section 452 of the conducting wire 450. The pump case 410 has a connecting member 415 configured to be connected to the cover 470. The connecting member 415 surrounds the first wire hole 413, the second wire hole 414, and the isolation section 452. In other words, the connecting member 415 defines a region on the casing wall of the pump case 410. The first wire hole 413, the second wire hole 414, and the isolation section 452 are located inside this region defined by the connecting member 415. In some embodiments, the cover 470 is connected to the connecting member 415 by welding.

In order to prevent damage to portions of the conducting wire 450 located at the first wire hole 413 and the second wire hole 414 and damage to the isolation section 452 located outside the accommodation cavity 412 caused by heat generated during the cover 470 being mounted to the pump case 410, the first wire hole 413, the second wire hole 414, and the isolation section 452 of the conducting wire 450 are all spaced apart from the connecting member 415 by a distance, so that the conducting wire 450 is kept at a distance from the connecting member 415 of the pump case 410 configured to be connected to the cover 470, to prevent damage to the conducting wire 450 due to heat generated during the cover 470 being connected to the pump case 410, and for example, a high temperature may damage an insulation layer of the conducting wire 450. In addition, both the first wire hole 413 and the second wire hole 414 being spaced apart from the connecting member 415 by a distance can also reduce the amount of the heat generated during mounting the cover 470 to the pump case 410 entering into the accommodation cavity 412 from the first wire hole 413 and the second wire hole 414, thereby reducing damage to elements in the accommodation cavity 412 caused by the heat during the mounting of the cover 470, for example, damage to the insulation layer of the coil or the effect on magnetism of components having magnetic properties. Therefore, with the above configuration, a yield can be increased, and failures of the blood pump 10 can be reduced.

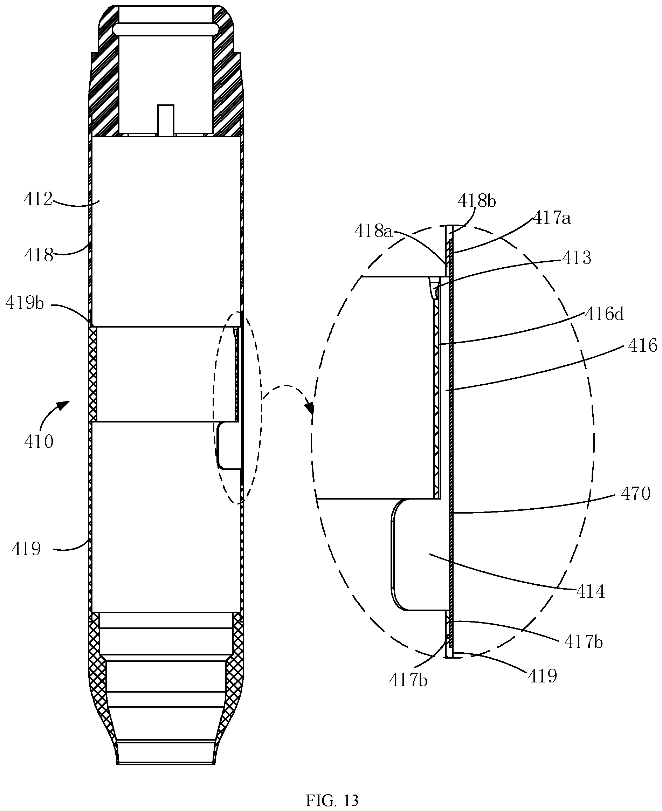

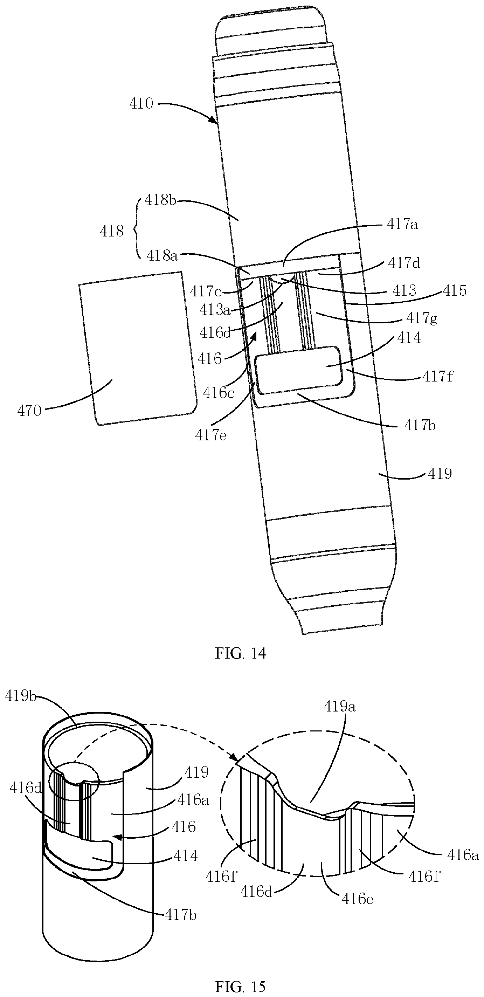

Referring to FIG. 13 and FIG. 14 together, in the illustrated embodiments, an outer side surface of the pump case 410 forms a sinking groove 416. The sinking groove 416 has a bottom wall 416a and an opening opposite to the bottom wall 416a. The cover 470 is at least partially received in the sinking groove 416. An edge of the cover 470 is connected to the connecting member 415. The cover 470 seals the opening of the sinking groove 416. The connecting member 415 is disposed in a circle around the opening of the sinking groove 416. Specifically, the connecting member 415 is an edge of the opening of the sinking groove 416. Both the first wire hole 413 and the second wire hole 414 are disposed on the bottom wall 416a of the sinking groove 416. Both the first wire hole 413 and the second wire hole 414 are spaced apart from an edge of the bottom wall 416a of the sinking groove 416 by a distance. The isolation section 452 of the conducting wire 450 is located between the bottom wall 416a of the sinking groove 416 and the cover 470. The bottom wall 416a of the sinking groove 416 is located between the rotor 440 and the isolation section 452 of the conducting wire 450.

The isolation section 452 of the conducting wire 450 is isolated by recessing an outer peripheral side surface of the pump case 410 to form the sinking groove 416, and at least part of the cover 470 is received in the sinking groove 416, which can reduce an increase in a local outer diameter of the pump case 410 due to the arrangement of the cover 470 and even may not increase the local outer diameter of the pump case 410. Moreover, the sinking groove 416 can limit the cover 470, which facilitates the mounting of the cover 470 and can also better ensure stability of the connection between the cover 470 and the pump case 410.

Specifically, the sinking groove 416 further has a sidewall 416c disposed in a circle around the bottom wall 416a. The sidewall 416c of the sinking groove 416 is substantially perpendicular to the bottom wall 416a. Alternatively, an angle formed between the sidewall 416c of the sinking groove 416 and the bottom wall 416a is greater than 90° and less than 180°.

In the illustrated embodiments, the first wire hole 413 and the second wire hole 414 are spaced apart in the axial direction of the pump case 410. The first wire hole 413 is closer to the connecting end 421 of the rotating shaft 420 than the second wire hole 414. In a circumferential direction of the pump case 410, widths of the first wire hole 413 and the second wire hole 414 are both less than a width of the bottom wall 416a of the sinking groove 416. In the axial direction of the pump case 410, a maximum distance between sides of the first wire hole 413 and the second wire hole 414 away from each other is less than the width of the bottom wall 416a of the sinking groove 416, so that the first wire hole 413 and the second wire hole 414 are both spaced apart from a boundary of the bottom wall 416a of the sinking groove 416 by a distance. Therefore, in the axial direction of the pump case 410, a distal isolation portion 417a is formed between the first wire hole 413 and a distal boundary of the bottom wall 416a of the sinking groove 416, and a proximal isolation portion 417b is formed between the second wire hole 414 and a proximal boundary of the bottom wall 416a of the sinking groove 416. In the circumferential direction of the pump case 410, a first side isolation portion 417c and a second side isolation portion 417d are respectively formed between the first wire hole 413 and boundaries of two sides of the bottom wall 416a of the sinking groove 416, a third side isolation portion 417e and a fourth side isolation portion 417f are formed between the second wire hole 414 and the boundaries of the two sides of the bottom wall 416a of the sinking groove 416. A portion of the bottom wall 416a located between the first wire hole 413 and the second wire hole 414 forms a blocking portion 417g. The distal isolation portion 417a, the proximal isolation portion 417b, the first side isolation portion 417c, the second side isolation portion 417d, the third side isolation portion 417e, the fourth side isolation portion 417f, and the blocking portion 417g are connected to jointly form the bottom wall 416a of the sinking groove 416. The blocking portion 417g is located between the isolation section 452 of the conducting wire 450 and the rotor 440.

It is to be noted that in the present application, a side of the pump case 410 adjacent to the impeller 200 is defined as a distal end, and an end of the pump case 410 away from the impeller 200 is defined as a proximal end. Correspondingly, a distal boundary of the bottom wall 416a of the sinking groove 416 is a boundary of the bottom wall 416a at a side adjacent to the impeller 200, and a proximal boundary of the bottom wall 416a of the sinking groove 416 is a boundary of the bottom wall 416a at a side away from the impeller 200. A direction in which the pump case 410 goes around the rotating shaft 420 in a circle is defined as the circumferential direction of the pump case 410.

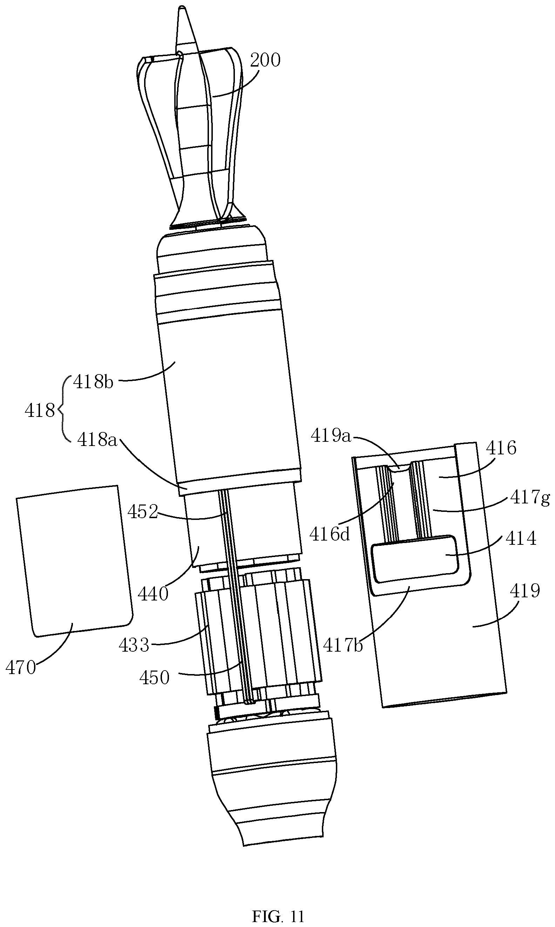

Referring to FIG. 11 to FIG. 15, specifically, the pump case 410 includes a first casing 418 and a second casing 419 connected to the first casing 418. The second casing 419 is connected to the first casing 418 to jointly define the accommodation cavity 412. The first casing 418 is closer to the connecting end 421 of the rotating shaft 420 than the second casing 419. The sinking groove 416 has one part located on the second casing 419 and another part located on the first casing 418. The second wire hole 414 is located on the second casing 419. The second casing 419 is provided with a notch 419a. The notch 419a is at least part of the first wire hole 413. At least one of the first casing 418 and the second casing 419 is provided with the notch, and then the first wire hole 413 is formed by connecting the first casing 418 and the second casing 419, so that the conducting wire 450 extending into the first wire hole 413, especially into the first wire hole 413 with a smaller hole diameter, during the assembling can be facilitated, thereby improving assembling efficiency. The first stator member 432 is received in the first casing 418. The second stator member 433 and the rotor 440 are received in the second casing 419. In the illustrated embodiments, the distal isolation portion 417a is located on the first casing 418; and the proximal isolation portion 417b, the first side isolation portion 417c, the second side isolation portion 417d, the third side isolation portion 417e, the fourth side isolation portion 417f, and the blocking portion 417g are all located on the second casing 419.

Specifically, an inner wall of the second casing 419 adjacent to the first casing 418 is provided with a step portion 419b. The first casing 418 includes a first section 418a and a second section 418b that are arranged coaxially. An outer diameter of the first section 418a is less than an outer diameter of the second section 418b. the first section 418a is inserted into the second casing 419. An end surface oft an end of the first section 418a away from the second section 418b abuts against the step portion 419b of the second casing 419, and the second section 418b is located outside the second casing 419. With the above configuration, during the mounting, the first section 418a of the first casing 418 is inserted into the second casing 419, at least one of the second casing 419 and the first casing 418 can be rotated to adjust the mounting position; and the second section 418b of the first casing 418 is located outside the second casing 419 and abuts against the end of the second casing 419. In this way, the second casing 419 can be fully utilized to position and guide the first casing 418, and the assembling efficiency and assembling accuracy between the second casing 419 and the first casing 418 can be improved. The sinking groove 416 has a part located on the first section 418a and another part located on the second casing 419. More specifically, the distal isolation portion 417a is located on the first section 418a.

It is to be noted that the pump case 410 is not limited to being separate. In some embodiments, the pump case 410 may be integrally formed. The first wire hole 413 and/or the second wire hole 414 is/are not limited to have the above configuration. In some embodiments, the first wire hole 413 may be completely disposed on the first casing 418 or the second casing 419. In some embodiments, an end of the second casing 419 adjacent to the first casing 418 is also provided with a notch. The notch of the second casing 419 is positioned corresponding to the notch of the first casing 418. The notch of the second casing 419 and the notch of the first casing 418 jointly form the first wire hole 413. In some embodiments, an end of the first casing 418 adjacent to the second casing 419 is provided with the notch, the second casing 419 is provided with no notch, so that after the first casing 418 is connected to the second casing 419, the second wire hole 414 is formed at the notch.

In the illustrated embodiments, a shape and a size of the cover 470 match those of the sinking groove 416. An outer side surface of the cover 470 is coplanar with the outer side surface of the pump case 410, so that the arrangement of the cover 470 may not increase the local outer diameter of the pump case 410. Specifically, the cover 470 is generally in the shape of an arc plate, and the outer peripheral side surface of the pump case 410 and the outer surface of the cover 470 transition smoothly.

Specifically, the edge of the cover 470 abuts against the edge of the bottom wall 416a of the sinking groove 416, so that the bottom wall 416a can support the cover 470 when the cover 470 is mounted, so as to facilitate the mounting of the cover 470 onto the pump case 410. More specifically, the cover 470 abuts against two sides of each of the proximal isolation portion 417b, the distal isolation portion 417a, the first side isolation portion 417c, the second side isolation portion 417d, the third side isolation portion 417e, the fourth side isolation portion 417f, and the blocking portion 417g of the bottom wall 416a in the circumferential direction of the pump case 410.

Specifically, a hole wall of at least one of the first wire hole 413 and the second wire hole 414 on a side adjacent to the other of the first wire hole 413 and the second wire hole 414 is an arc-shaped wall. The arc-shaped wall is a concave wall. In this way, a probability that the hole wall of the first wire hole 413 and/or the second wire hole 414 scratches the insulation layer of the conducting wire 450 can be reduced. In the illustrated embodiments, a hole wall 413a of the first wire hole 413 at a side adjacent to the second wire hole 414 is an arc-shaped wall.

In the illustrated embodiments, in the circumferential direction of the pump case 410, the width of the first wire hole 413 is less than the width of the second wire hole 414. In the case of a plurality of conducting wires 450, the smaller first wire hole 413 can have an effect of gathering and concentrating the isolation sections 452 of the plurality of conducting wires 450, and the larger second wire hole 414 can facilitate separate wiring of the conducting wires 450 into the accommodation cavity 412, so as to facilitate reasonable arrangement of the wiring of the conducting wires 450.

In an embodiment, the casing wall of the pump case 410 is provided with a limiting groove 416d. The limiting groove 416d is located on an outer side of the casing wall of the pump case 410. The limiting groove 416d is located between the first wire hole 413 and the second wire hole 414. In the illustrated embodiments, the limiting groove 416d is formed by locally recessing a surface of the bottom wall 416a (specifically, the blocking portion 417g) of the sinking groove 416 facing the cover 470. In the circumferential direction of the pump case 410, a width of the bottom wall 416a (specifically, the blocking portion 417g) of the sinking groove 416 is greater than a width of the limiting groove 416d. Therefore, in the circumferential direction of the pump case 410, the width of the limiting groove 416d is also less than a width of the region defined by the connecting member 415. In other words, the connecting member 415 is disposed in a circle around the first wire hole 413, the second wire hole 414, and the limiting groove 416d. The cover 470 also completely covers the limiting groove 416d. At least part of the isolation section 452 of the conducting wire 450 is limited in the limiting groove 416d. The limiting groove 416d has a limiting effect on the isolation section 452 of the conducting wire 450. Then, when the driving device 400 is assembled, especially when the cover 470 is connected to the pump case 410, the conducting wire 450 between the first wire hole 413 and the second wire hole 414 can be limited, which can prevent inconvenience in the mounting of the cover 470 due to scattering or movement of the conducting wires 450, or prevent the conducting wire 450 between the first wire hole 413 and the second wire hole 414 from moving to a junction (i.e., the connecting member 415) between the cover 470 and the pump case 410 when the cover 470 is mounted, thereby further ensuring that the isolation section 452 of the conducting wire 450 can be away from the connecting member 415 of the pump case 410 connected to the cover 470.

In the illustrated embodiments, the limiting groove 416d extends in the same direction as the axial direction of the pump case 410. In the circumferential direction of the pump case 410, the width of the limiting groove 416d is greater than or equal to the width of the first wire hole 413 and less than the width of the second wire hole 414, so as to facilitate the isolation sections 452 of the conducting wires 450 to be collected into the limiting groove 416d and also facilitate the conducting wires 450 to be wired separately at the second wire hole 414 and into the accommodation cavity 412.

Specifically, the limiting groove 416d has an end in communication with the first wire hole 413 and another end in communication with the second wire hole 414, so that the hole wall of the first wire hole 413 at a side adjacent to the limiting groove 416d is concave compared with the surface of the bottom wall 416a facing the cover 470, and the hole wall of the second wire hole 414 at a side adjacent to the limiting groove 416d is also concave compared with the surface of the bottom wall 416a facing the cover 470, thereby reducing bending radians of the conducting wire 450 at the first wire hole 413 and the second wire hole 414 and reducing damage to the conducting wire 450 by the hole walls of the first wire hole 413 and the second wire hole 414.

In the embodiments illustrated, in the circumferential direction of the pump case 410, the limiting groove 416d has a middle portion 416e with a larger recess depth and two side portions 416f located on two sides of the middle portion 416e and with smaller recess depths. A thickness of the casing wall of the pump case 410 at the middle portion 416e of the limiting groove 416d is less than thicknesses of the casing wall of the pump case 410 at the two side portions 416f of the limiting groove 416d. The recess depth of each side portion 416f gradually decreases relative to the surface of the bottom wall 416a facing the cover 470 in a direction away from the middle portion 416e. That is, in the direction away from the middle portion 416e, the thickness of the casing wall of the pump case 410 at each side portion 416f gradually increases. In this way, processing and molding of the pump case 410 can be facilitated, and a yield of the pump case 410 can be increased. A groove wall of the side portion 416f of the limiting groove 416d is arc-shaped, to reduce damage to the conducting wire 450.

Specifically, a width of the middle portion 416e of the limiting groove 416d is greater than or equal to a width of sequentially arranging the isolation sections 452 of the plurality of conducting wires 450 connected to the first stator member in parallel, to reduce the depth of the limiting groove 416d as much as possible, so that the casing wall of the pump case 410 can have a thickness as large as possible at a position corresponding to the limiting groove 416d, to give the pump case 410 as much strength as possible.

It is to be noted that in some embodiments, the sinking groove 416 of the casing wall of the pump case 410 may be omitted, and the limiting groove 416d is directly provided on the outer side of the casing wall of the pump case 410. In this case, the limiting groove 416d may be formed by locally recessing the outer surface of the casing wall of the pump case 410; and a groove wall of the limiting groove 416d is located between the rotor 440 and the isolation section 452, to separate the isolation section 452 of the conducting wire 450 from the rotor 440. The cover 470 is connected to the pump case 410 and completely covers the first wire hole 413, the second wire hole 414, the limiting groove 416d, and the isolation section 452 to prevent entry of foreign objects (such as blood) into the accommodation cavity 412 through the first wire hole 413 and the second wire hole 414 and also prevent exposure of the isolation section 452 of the conducting wire 450. Correspondingly, the connecting member 415 is disposed in a circle around the first wire hole 413, the second wire hole 414, and the limiting groove 416d, and the first wire hole 413, the second wire hole 414, and the limiting groove 416d are all spaced apart from the connecting member 415 by a distance, to prevent damage to the conducting wire 450 due to heat generated during connecting the cover 470 to the pump case 410, and for example, a high temperature may damage the insulation layer of the conducting wire 450. In addition, both the first wire hole 413 and the second wire hole 414 being spaced apart from the connecting member 415 by a distance can also reduce the amount of the heat generated during mounting the cover 470 to the pump case 410 entering the accommodation cavity 412 from the first wire hole 413 and the second wire hole 414, thereby reducing damage to the components in the accommodation cavity 412 caused by the heat during the mounting of the cover 470, for example, damage to the insulation layer of the coil or the effect on magnetism of components having magnetic properties, so as to increase the yield of the driving device 400 and reduce failures.

Referring to FIG. 3 and FIG. 4, the driving device 400 further includes a shaft sleeve assembly 480. The shaft sleeve assembly 480 is fixed in the pump case 410. The shaft sleeve assembly 480 is located between the first stator member 432 and the impeller 200. The rotating shaft 420 rotatably extends through the shaft sleeve assembly 480. Through the arrangement of the shaft sleeve assembly 480, the rotating shaft 420 can be radially limited, reducing radial shaking of the rotating shaft 420 during the rotation, and improving stability of the rotation of the rotating shaft 420. The shaft sleeve assembly 480 may be integrally formed or separately formed. In the illustrated embodiments, the pump case 410 further has a third casing 405. The third casing 405 is connected to an end of the first casing 408 away from the second casing 419. The shaft sleeve assembly 410 is fixedly received in the third casing 405.

In the illustrated embodiments, the shaft sleeve assembly 480 includes a first shaft sleeve 482 and a second shaft sleeve 484. Both the first shaft sleeve 482 and the second shaft sleeve 484 are fixedly connected to the pump case 410. The rotating shaft 420 rotatably extends through the first shaft sleeve 482 and the second shaft sleeve 484. The first shaft sleeve 482 is closer to the impeller 200 than the second shaft sleeve 484.

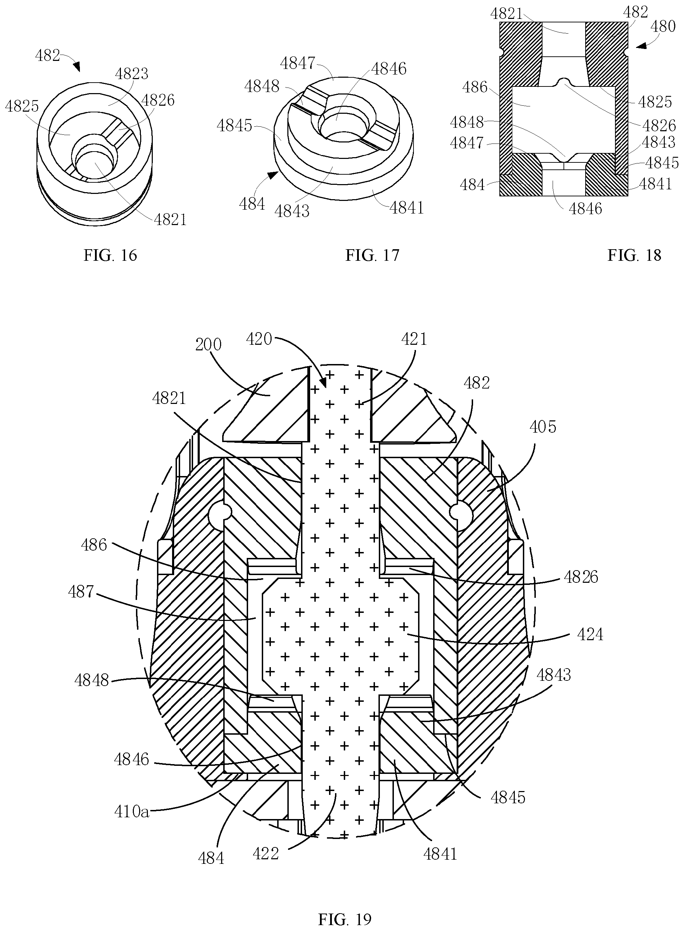

Referring to FIG. 16 together, the first shaft sleeve 482 is provided with a first through hole 4821 and a limiting hole 4823. The first through hole 4821 and the limiting hole 4823 are in communication with each other and arranged coaxially. A hole diameter of the limiting hole 4823 is greater than a hole diameter of the first through hole 4821, so that the first through hole 4821 and the limiting hole 4823 jointly form a step hole. The first shaft sleeve 482 has a first limiting surface 4825 that defines part of a boundary of the limiting hole 4823.

Referring to FIG. 17 together, the second shaft sleeve 484 includes a thick section 4841 and a thin section 4843. A cross section of the thin section 4843 is smaller than a cross section of the thick section 4841, and the thick section 4841 has an abutment surface 4845. The thin section 4843 is disposed on and protrude from the abutment surface 4845. The second shaft sleeve 484 has a second through hole 4846. The second through hole 4846 extends from an end surface of an end of the thin section 4843 away from the abutment surface 4845 to a side of the thick section 4841 away from the abutment surface 4845, so that the second through hole 4846 runs through the thick section 4841 and the thin section 4843.

Referring to FIG. 18 together, the thick section 4843 is inserted into the limiting hole 4823, and the abutment surface 4845 abuts against the first shaft sleeve 482. Through a limiting function of the abutment surface 4845 and a guiding function of the thin section 4843, mounting accuracy and mounting efficiency of the entire shaft sleeve assembly 480 can be improved. The end surface of the end of the thin section 4843 away from the abutment surface 4845 is a second limiting surface 4847. The second limiting surface 4847 and the first limiting surface 4825 are spaced apart and face each other. The hole wall of the limiting hole 4823, the first limiting surface 4825, and the second limiting surface 4847 jointly define a limiting cavity 486. The first through hole 4821 and the second through hole 4846 are both in communication with the limiting cavity 486.

Referring to FIG. 19 together, the rotating shaft 420 includes a straight shaft portion 422 and an annular raised portion 424. The annular raised portion 424 is fixedly sleeved on the straight shaft portion 422. An outer diameter of the annular raised portion 424 is greater than a diameter of the straight shaft portion 422.

The straight shaft portion 422 extends through the first through hole 4821 and the second through hole 4846. The annular raised portion 424 is received in the limiting cavity 486. The outer diameter of the annular raised portion 424 is greater than the hole diameter of the first through hole 4821, and the outer diameter of the annular raised portion 424 is greater than the hole diameter of the second through hole 4846. The straight shaft portion 422 rotatably extends through the first stator member 432. The rotor 440 is fixedly connected to the straight shaft portion 422. The connecting end 421 is an end of the straight shaft portion 422.

In the extending direction of the rotating shaft 420, the annular raised portion 424 is located between the first limiting surface 4825 and the second limiting surface 4847. The annular raised portion 424 can abut against the first limiting surface 4825 and the second limiting surface 4847 to position the rotating shaft 420 in the axial direction of the pump case 410, thereby preventing movement of the rotating shaft 420 in the axial direction of the pump case 410 or limiting a range of the movement of the rotating shaft 420 in the axial direction of the pump case 410. In some embodiments, the annular raised portion 424 always abuts against the first limiting surface 4825 and the second limiting surface 4847. In some embodiments, a distance between the first limiting surface 4825 and the second limiting surface 4847 is slightly greater than an axial height of the annular raised portion 424, so that during the rotation of the rotating shaft 420, the annular raised portion 424 has a certain floating space between the first limiting surface 4825 and the second limiting surface 4847 for flow of cleaning fluid.

A first gap configured for the flow of the cleaning fluid is formed between the straight shaft portion 422 and the hole wall of the first through hole 4821. A second gap configured for the flow of the cleaning fluid is formed between the straight shaft portion 422 and the hole wall of the second through hole 4846. A third gap 487 configured for the flow of the cleaning fluid is formed between the annular raised portion 424 and a cavity wall of the limiting cavity 486.

The first limiting surface 4825 is partially recessed to form a first guide groove 4826. The first guide groove 4826 is in communication with the first through hole 4821. The first guide groove 4826 is in communication with the limiting cavity 486. The second limiting surface 4847 is partially recessed to form a second guide groove 4848. The second guide groove 4848 is in communication with the second through hole 4846. The first guide groove 4826 is in communication with the limiting cavity 486. In this way, when the annular raised portion 424 abuts against the first limiting surface 4825 and/or the second limiting surface 4847, the first guide groove 4826 can be in communication with the first gap and the third gap 487, and the second guide groove 4848 can be in communication with the second gap and the third gap 487. The first through hole 4821 is in communication with the cannula assembly 100, and the second through hole 4846 is in communication with the accommodation cavity 412 of the pump case 410.

When the cleaning fluid is introduced into an end of the blood pump 10 away from the cannula assembly 100, the liquid may flow through the accommodation cavity 412, the second gap, the third gap 487, and the first gap in sequence, then enter the cannula assembly 100, and then flow out from the outlet 120 of the cannula assembly 100. Since a flow direction of the cleaning fluid is opposite to a flow direction of the blood in the cannula assembly 100, the blood in the cannula assembly 100 can be prevented from entering the driving device 400. Moreover, the injected cleaning fluid flows in the first gap, the second gap, and the third gap 487, which also acts as a lubricant between the rotating shaft 420 and the shaft sleeve assembly 480, reducing rotational resistance of the rotating shaft 420.

Referring to FIG. 19, the pump case 410 is further provided with a bearing protrusion 410a. The bearing protrusion 410a is annular. The side of the thick section 4841 of the second shaft sleeve 484 in the shaft sleeve assembly 480 away from the abutment surface 4845 abuts against the bearing protrusion 410a. The bearing protrusion 410a limits the entire shaft sleeve assembly 480 in the axial direction of the pump case 410. Specifically, the bearing protrusion 410a is located on the third casing 405. The first back plate 4323 of the first stator member 432 is fixedly connected to the third casing 405.

It is to be noted that the driving device 400 is not limited to have the above configuration. In some embodiments, the rotor 440 is still located between the first stator member 432 and the second stator member 433, the rotor 440 has two flywheels fixedly connected to the rotating shaft 420. The first magnet 442 and the second magnet 443 are respectively mounted on the two flywheels.

In some embodiments, the rotor 440 may also include only the first magnet 442 and the second magnet 443 without the flywheel 444. That is, the rotor 440 is still located between the first stator member 432 and the second stator member 433. In this case, the first magnet 442 and the second magnet 443 are directly fixedly connected to the rotating shaft 420. In this case, the position of the isolation section 452 of the conducting wire 450 corresponds to positions of both the first magnet 442 and the second magnet 443.

In some embodiments, the first magnet 442, the first stator member 432, the second magnet 443, and the second stator member 433 are arranged in sequence in the extending direction of the rotating shaft 420. The rotating shaft 420 may extend through only the first stator member 432. The rotating shaft 420 may also extend through the first stator member 432 and the second stator member 433, and the first magnet 442 is adjacent to the connecting end 421 of the rotating shaft 420. In this case, the position of the isolation section 452 of the conducting wire 450 corresponds to the position of the second magnet 443.

In some embodiments, the first magnet 442, the first stator member 432, the second stator member 433, and the second magnet 443 are arranged in sequence in the extending direction of the rotating shaft 420. The rotating shaft 420 rotatably extends through the first stator member 432 and the second stator member 433, and the first magnet 442 is adjacent to the connecting end 421 of the rotating shaft 420. In this case, a plurality of conducting wires 450 are provided, part of the conducting wires 450 are connected to the first stator member 432, part of the conducting wires 450 are connected to the second stator member 433. Each conducting wire 450 has an isolation section 452. Positions of the isolation sections 452 of the plurality of conducting wires 450 each correspond to the position of the second magnet 443. In this case, the plurality of conducting wires 450 all extends through the first wire hole 413 and the second wire hole 414 so that the isolation section 452 of each conducting wire 450 is located outside the accommodation cavity 412. Alternatively, the first stator member 432 and the second stator member 433 are connected through another conducting wire; and the conducting wire 450 is connected to one of the first stator member 432 and the second stator member 433.

In some embodiments, the first stator member 432, the first magnet 442, the second stator member 433, and the second magnet 443 are arranged in sequence in the extending direction of the rotating shaft 420; and the first stator member 432 is adjacent to the connecting end 421 of the rotating shaft 420. In this case, the pump case 410 may be provided with both the first wire hole 413 and the second wire hole 414 at positions corresponding to the first magnet 442 and the second magnet 443, to isolate the isolation section 452 of the conducting wire 450.

In some embodiments, the rotor 440 has only a magnet. In this case, the first stator member 432 and the second stator member 433 share one magnet, and the rotor 440 is still located between the first stator member 432 and the second stator member 433.

In some embodiments, the stator 420 has only one stator member. In this case, if the impeller 200, the stator 430, and the rotor 440 are arranged in sequence in the extending direction of the rotating shaft 420, the conducting wire 450 is connected to the stator 430.

Since the driving device in this embodiment has a configuration similar to the driving device in the first embodiment, the driving device in this embodiment and the blood pump having the driving device in this embodiment also have a similar effect to that of the first embodiment.

The above embodiments are merely intended for describing the technical solutions of the present application rather than limiting the present application. Although the present application is described in detail with reference to the above embodiments, those of ordinary skill in the art should understand that they may still make modifications to the technical solutions described in the above embodiments or make equivalent replacements to some technical features thereof, and such modifications or replacements do not cause the essence of the corresponding technical solutions to depart from the spirit and scope of the technical solutions in the embodiments of the present application, all of which should fall within the protection scope of the present application.

## Claims

1. A driving device configured to drive an impeller to rotate, wherein the driving device comprises:
a pump case having an accommodation cavity, a casing wall of the pump case being further provided with a first wire hole, a second wire hole, and a limiting groove, the second wire hole and the first wire hole being in communication with the accommodation cavity, the second wire hole being spaced apart from the first wire hole, the limiting groove being located on an outer side of the casing wall of the pump case, and the limiting groove being located between the first wire hole and the second wire hole;
a rotating shaft configured to be connected to the impeller, the rotating shaft being rotatably mounted on the pump case;
a rotor rotatably received in the accommodation cavity, the rotor being fixedly connected to the rotating shaft, and the rotor being capable of driving the rotating shaft to rotate;
a stator received in the accommodation cavity, the stator being capable of driving the rotor to rotate;
a conducting wire connected to the stator, the conducting wire extending through the first wire hole and the second wire hole, the conducting wire having an isolation section corresponding to a position of the rotor, the isolation section being located between the first wire hole and the second wire hole, the isolation section being at least partially limited in the limiting groove, and a groove wall of the limiting groove being located between the rotor and the isolation section; and
a cover connected to the pump case and completely covering the first wire hole, the second wire hole, the limiting groove, and the isolation section.

2. The driving device according to claim 1, wherein the limiting groove extends from the first wire hole to the second wire hole; the first wire hole and the second wire hole are in communication with the limiting groove; and an outer side surface of the pump case is recessed to form the limiting groove.

3. The driving device according to claim 2, wherein in a circumferential direction of the pump case, the limiting groove comprise: a middle portion with a larger recess depth; and two side portions located on two sides of the middle portion and having smaller recess depths, wherein a thickness of the casing wall of the pump case at the middle portion of the limiting groove is less than thicknesses of the casing wall of the pump case at the two side portions of the limiting groove, and in a direction away from the middle portion, the thickness of the casing wall of the pump case at each of the side portions gradually increases.

4. The driving device according to claim 2, wherein a plurality of conducting wires are provided, each of the conducting wires has the isolation section (452); and a width of a middle portion of the limiting groove is greater than or equal to widths of sequentially arranging the isolation sections of the plurality of conducting wires in parallel.

5. The driving device according to claim 1, wherein in a circumferential direction of the pump case, a width of at least one of the first wire hole and the second wire hole is greater than a width of the limiting groove.

6. The driving device according to claim 1, wherein in a circumferential direction of the pump case, a width of one of the first wire hole and the second wire hole is equal to or less than a width of the limiting groove; or in the circumferential direction of the pump case, the width of one of the first wire hole and the second wire hole is less than or equal to the width of the limiting groove, and a width of another of the first wire hole and the second wire hole is greater than the width of the limiting groove.

7. The driving device according to claim 1, wherein a hole wall of at least one of the first wire hole and the second wire hole at a side adjacent to the limiting groove is an arc-shaped wall, the arc-shaped wall being a concave wall.

8. The driving device according to claim 1, wherein the pump case is provided with a connecting member, the connecting member being disposed in a circle around the first wire hole, the second wire hole, and the limiting groove; the first wire hole, the second wire hole, and the limiting groove are spaced apart from the connecting member by a distance; and the cover is connected to the connecting member.

9. The driving device according to claim 8, wherein an outer side surface of the pump case is recessed to form a sinking groove, the sinking groove having a bottom wall and an opening opposite to the bottom wall; the first wire hole, the second wire hole, and the limiting groove are located on the bottom wall of the sinking groove; in a circumferential direction of the pump case, a width of the bottom wall of the sinking groove is greater than a width of the limiting groove; the cover is at least partially received in the sinking groove; and the connecting member is an edge of the opening of the sinking groove.

10. The driving device according to claim 9, wherein the first wire hole, the second wire hole, and the limiting groove are spaced apart from an edge of the bottom wall of the sinking groove by a distance; and an edge of the cover abuts against the edge of the bottom wall of the sinking groove.

11. The driving device according to claim 9, wherein the pump case comprises a first casing and a second casing connected to the first casing, the second casing being connected to the first casing to jointly define the accommodation cavity; and the sinking groove has a part located on the second casing and another part located on the first casing; an inner wall of the second casing adjacent to the first casing is provided with a step portion; the first casing comprises a first section and a second section that are arranged coaxially, wherein an outer diameter of the first section is less than an outer diameter of the second section; the first section is inserted into the second casing; an end surface of an end of the first section away from the second section abuts against the step portion of the second casing; and the second section is located outside the second casing; wherein the bottom wall of the sinking groove has a part located on the first section and another part located on the second casing.

12. The driving device according to claim 8, wherein the cover is connected to the connecting member by welding; or an outer side surface of the pump case is recessed to form a sinking groove, the sinking groove having a bottom wall and an opening opposite to the bottom wall, the bottom wall being provided with the limiting groove, and wherein a side of the cover facing away from the bottom wall of the sinking groove is coplanar with an outer side surface of the pump case.

13. The driving device according to claim 1, wherein the pump case comprises a first casing and a second casing connected to the first casing, the second casing being connected to the first casing to jointly define the accommodation cavity, wherein the second wire hole is located on the second casing, an end portion of at least one of the second casing and the first casing adjacent to another of the second casing and the first casing is provided with a notch, the notch being at least part of the first wire hole; and the limiting groove is located on the second casing; and in a circumferential direction of the pump case, a width of the first wire hole is less than a width of the second wire hole.

14. The driving device according to claim 1, wherein the rotor is magnetic; and the stator comprises a first stator member and a second stator member that are arranged in an extending direction of the rotating shaft, the first stator member and the second stator member being capable of generating a rotating magnetic field that drives the rotor to rotate; wherein the first stator member and the second stator member are fixedly connected to the pump case; the rotating shaft rotatably extends through the first stator member and is spaced apart from the second stator member; and the first stator member and the second stator member each have a magnetic core, wherein the magnetic core comprises a magnetic post; and a cross section of the magnetic post of the second stator member is greater than a cross section of the magnetic post of the first stator member.

15. The driving device according to claim 14, wherein the rotor comprises a first magnet and a second magnet, the first magnet and the second magnet being fixedly connected to the rotating shaft; in an extending direction of the rotating shaft, the second magnet is located between the first stator member and the second stator member; the first stator member is capable of generating a rotating magnetic field that drives the first magnet to rotate, and the second stator member is capable of generating a rotating magnetic field that drives the second magnet to rotate; and the conducting wire is connected to the first stator member; and a position of the isolation section at least corresponds to a position of the second magnet.

16. The driving device according to claim 14, wherein the rotor comprises a flywheel, a first magnet, and a second magnet, the flywheel being fixedly connected to the rotating shaft, the flywheel being located between the first stator member and the second stator member, the first magnet and the second magnet being mounted on the flywheel; the first stator member is capable of generating a rotating magnetic field that drives the first magnet to rotate, and the second stator member is capable of generating a rotating magnetic field that drives the second magnet to rotate.

17. The driving device according to claim 16, wherein the flywheel comprises a disc-shaped portion; and the rotating shaft fixedly extends through the disc-shaped portion; the first magnet and the second magnet are respectively disposed on two opposite sides of the disc-shaped portion; the first magnet and the second magnet are annular Halbach array magnets; the first magnet comprises a first magnetic body magnetized in an axial direction of the first magnet; the second magnet comprises a second magnetic body magnetized in an axial direction of the second magnet; wherein the first magnetic body and the second magnetic body are positioned facing to each other, and in an extending direction of the rotating shaft, the first magnetic body and the second magnetic body have opposite polarities on a respective side thereof facing the disc-shaped portion.

18. The driving device according to claim 1, wherein the rotating shaft comprises a connecting end configured to be connected to the impeller; the rotor comprises a first magnet and a second magnet, the first magnet and the second magnet being fixedly connected to the rotating shaft; and the stator comprises a first stator member and a second stator member that are arranged in an extending direction of the rotating shaft, the first stator member being capable of generating a rotating magnetic field that drives the first magnet to rotate, and the second stator member being capable of generating a rotating magnetic field that drives the second magnet to rotate; in the extending direction of the rotating shaft, the first magnet, the first stator member, the second stator member, and the second magnet are arranged sequentially in the extending direction of the rotating shaft, and the first magnet is adj acent to the connecting end of the rotating shaft; and at least one of the first stator member and the second stator member is connected to the conducting wire, and positions of the isolation section and the second magnet correspond to each other.

19. The driving device according to claim 1, wherein the stator comprises a first stator member and a second stator member that are arranged in an extending direction of the rotating shaft; the driving device further comprises a shaft sleeve assembly fixed in the pump case, the shaft sleeve assembly being located between the first stator member and the impeller; wherein the shaft sleeve assembly comprises a first shaft sleeve and a second shaft sleeve, the first shaft sleeve being provided with a first through hole, a limiting hole, and a first limiting surface, the second shaft sleeve being provided with a second through hole and a second limiting surface, and wherein a hole wall of the limiting hole, the first limiting surface, and the second limiting surface jointly defines a limiting cavity; and the rotating shaft comprises a straight shaft portion and an annular raised portion, the straight shaft portion extending through the first through hole and the second through hole, and the annular raised portion being received in the limiting cavity and located between the first limiting surface and the second limiting surface.

20. A blood pump, comprising an impeller and a driving device, the driving device being configured to drive the impeller to rotate, and the driving device comprising:
a pump case having an accommodation cavity, a casing wall of the pump case being further provided with a first wire hole, a second wire hole, and a limiting groove, the second wire hole and the first wire hole being in communication with the accommodation cavity, the second wire hole being spaced apart from the first wire hole, the limiting groove being located on an outer side of the casing wall of the pump case, and the limiting groove being located between the first wire hole and the second wire hole;
a rotating shaft configured to be connected to the impeller, the rotating shaft being rotatably mounted on the pump case;
a rotor rotatably received in the accommodation cavity, the rotor being fixedly connected to the rotating shaft, and the rotor being capable of driving the rotating shaft to rotate;
a stator received in the accommodation cavity, the stator being capable of driving the rotor to rotate;
a conducting wire connected to the stator, the conducting wire extending through the first wire hole and the second wire hole, the conducting wire having an isolation section corresponding to a position of the rotor, the isolation section being located between the first wire hole and the second wire hole, the isolation section being at least partially limited in the limiting groove, and a groove wall of the limiting groove being located between the rotor and the isolation section; and
a cover connected to the pump case and completely covering the first wire hole, the second wire hole, the limiting groove, and the isolation section;
wherein the impeller is fixedly connected to the rotating shaft, and the impeller is rotatable with the rotating shaft.
